(19) Europäisches Patentamt
European Patent Office
Office européen des brevets

(11) **EP 2 090 331 A1**

(12) **EUROPEAN PATENT APPLICATION**
published in accordance with Art. 153(4) EPC

(43) Date of publication:
**19.08.2009 Bulletin 2009/34**

(51) Int Cl.:
***A61M 37/00*** *(2006.01)*      ***B29C 43/02*** *(2006.01)*
***B29C 43/56*** *(2006.01)*

(21) Application number: **07832285.6**

(22) Date of filing: **21.11.2007**

(86) International application number:
**PCT/JP2007/072555**

(87) International publication number:
**WO 2008/062832 (29.05.2008 Gazette 2008/22)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.11.2006   JP 2006315371**

(71) Applicant: **Toppan Printing Co., Ltd.**
**Tokyo 110-0016 (JP)**

(72) Inventor: **TOMONO, Takao**
**Tokyo 110-0016 (JP)**

(74) Representative: **TBK-Patent**
**Bavariaring 4-6**
**80336 München (DE)**

(54) **MICRONEEDLE ARRAY AND PROCESS FOR PRODUCTION THEREOF**

(57)      [Problems] To provide a process by which non-discolored, shape-stable microneedles and microneedle arrays can be produced without impairing the sharpness of needle points in molding and while protecting the molecular weight from decrease due to hydrolysis, and products made by the process.

[Means for Solving Problems] A process for the production of microneedles which comprises the feeding step of feeding a resin fluid to a mold provided with needle forming sections having an opening diameter of 50 to 200 μm and a depth of 100 to 450 μm, the filling step of filling the resin fluid into the needle-forming sections, and the solidification step of solidifying the filled resin fluid by cooling, **characterized in that** the feeding, filling and solidification steps are conducted under a reduced pressure or vacuum.

FIG. 7

## Description

Technical Field

The present invention relates to a microneedle, a method for producing the microneedle, a microneedle array using the microneedle and a method for producing the microneedle array.

Background Art

Conventionally, for administration of a drug through the skin, the mucous membrane, or a like biological surface, usually, a liquid or gel drug is often applied. Although an application of a drug on a biological surface is a noninvasive method, the applied drug is easily removed by sweating, external contact, and the like. Further, when the administration is continued for a long period of time, a safety problem such as dermopathy may be caused. Further, when the subject drug has a large molecular weight, is water soluble, etc., such a drug is hardly absorbed into the body even if applied on a biological surface, and percutaneous administration thereof has thus been difficult.

In order to solve these problems, a microneedle array having a large number of 50 $\mu$m to 100 $\mu$m high microneedles provided on a substrate has been proposed (see, e.g., the patent document 1).

Although the method of administering a drug directly into the body tissue using a microneedle array having a desired drug applied to the surface of microneedles is not a perfectly noninvasive method, this seldom stimulates the sense of pain and is less invasive to the patient because microneedles have a small diameter and only reach the dermis or the like which is a region at a relatively shallow depth in the body tissue. Further, the drug can be administered in the state that the microneedles run through the epidermis and the horny layer, and this accordingly gives the advantage that drugs heretofore difficult to percutaneously administrate can also be administered. Patent document 1: Jpn. PCT National Publication No. 2005-503194

Disclosure of Invention

Problem to be solved by the Invention

The above microneedles are excellent in the puncturing ability as they are formed on a silicon single crystal substrate, however, there is a problem in that when the microneedles break, the residues remain in the skin.

An example of producing a needle shape with a degradable polymer, such as polylactic acid, has also been proposed. In such a case, however, because of the high aspect ratio, air in the tip of a needle-forming portion of a mold remains to cause a problem in the shape reproducibility.

The present invention was accomplished in view of the above background. An object thereof is to provide a production method for molding a microneedle and a microneedle array which do not obtain blunt needle tips at the time of molding, do not undergo hydrolysis, thus maintaining a stable molecular weight, do not suffer from coloring, and have excellent shape stability; and also to provide products therefrom.

Means for Solving Problem

A method for producing a microneedle of the present invention is characterized by comprising a feeding step of feeding a resin fluid to a forming mold having a needle-forming portion with an opening diameter of 50 to 200 $\mu$m and a depth of 100 to 450 $\mu$m, a charging step of charging the fed resin fluid into the needle-forming portion, and a solidifying step of cooling and solidifying the charged resin fluid, wherein the feeding step, the charging step, and the solidifying step are performed under reduced pressure or vacuum.

Another method for producing a microneedle of the present invention is characterized by comprising a feeding step of feeding a resin fluid to a forming mold having a needle-forming portion with an opening diameter of 50 to 200 $\mu$m and a depth of 100 to 450 $\mu$m, an overheating and melting step of fluidizing the fed resin to produce a resin fluid, a charging step of charging the resin fluid into the needle-forming portion, and a solidifying step of cooling and solidifying the charged resin fluid, wherein the feeding step, the overheating and melting step, the charging step, and the solidifying step are performed under reduced pressure or vacuum.

The forming mold may have two or more needle-forming portions. The forming mold may have a protruding portion in an area other than the needle-forming portion, or alternatively, may have a protruding portion in the needle-forming portion.

The step of charging the resin fluid may be performed by pressing the resin fluid.

In the step of charging the resin fluid, the resin fluid may be placed also in an area on the forming mold other than the needle-forming portion.

The resin may contain a biocompatible material, and the biocompatible material may be a material containing polylactic acid (PLA), glycol lactic acid, chitin, chitosan, hyaluronic acid, collagen, glucose/cellulose, or magnesium alloy.

A microneedle according to the present invention is **characterized in that** it is produced by either of the above producing methods. The microneedle may be the one that does not undergo plastic deformation under a load of 5 kg/cm$^2$ or less.

A method for producing a microneedle array according to the present invention is characterized by comprising a placing step of placing, after forming the microneedle, a substrate on the needle-forming portion of the forming mold, and a stacking step of integrating the needle-forming portion and the substrate together. The microneedle and the substrate may be made of different materials.

Effect of the Invention

According to the invention, molding is performed under reduced pressure or vacuum, and therefore, a sharp microneedle tip can be obtained. Further, since hydrolysis reaction is suppressed, reduction in the molecular weight is made less prone to occur and maintaining the strength of the microneedle is made possible. In addition, the suppression of reactions can avoid the problem of coloring.

Best Mode for Carrying Out the Invention

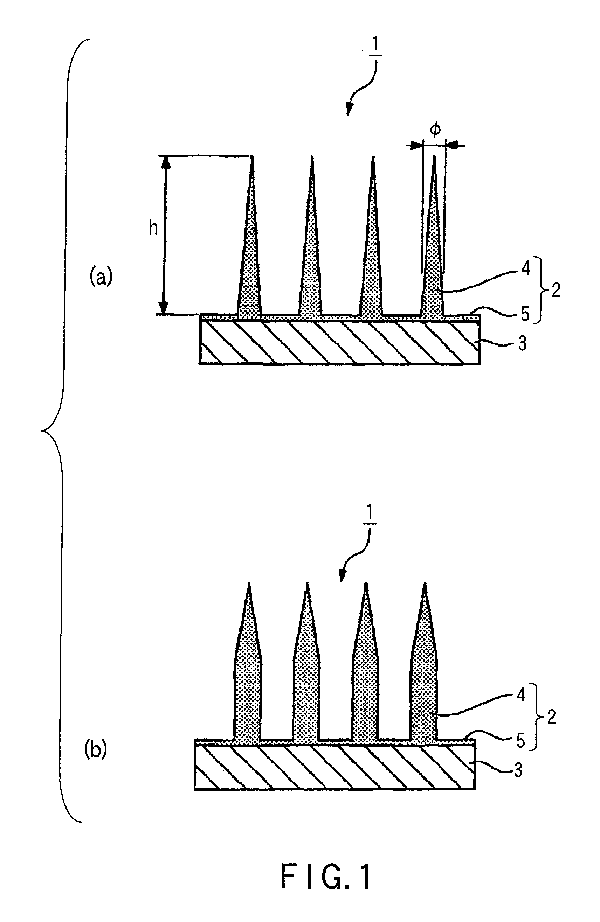

A microneedle array according to a first embodiment of the invention is explained with reference to FIG. 1 to FIG. 3.

As shown in FIG. 1 (a), a microneedle array 1 of this embodiment comprises a microneedle 2 and a substrate 3 which is provided beneath the microneedle 2 and supports the microneedle 2. The microneedle 2 is made of PLA, a biocompatible material, and comprises a large number of conical-shaped needle portions 4 integrally formed on a sheet portion 5. The substrate 3 is made of acryl (PMMA). The sheet portion 5 of the microneedle 2 is thermally fused to the surface of the substrate 3, thereby forming the microneedle array 1. The microneedle array 1 has microneedles formed at intervals of 200 to 1,200 microneedles/cm$^2$.

The needle portions 4 and the sheet portion 5 are made of medical-grade polylactic acid (PLA), a biocompatible material. The needle portions 4 have a bottom diameter $\varphi$ of 50 $\mu$m to 200 $\mu$m and a height h of 100 $\mu$m to 500 $\mu$m. In consideration of the balance between the degree of penetration of a drug applied to the surface and the degree of invasion due to the stimulation of the sense of pain, it is more preferable that the bottom diameter $\varphi$ be within the range of 80 $\mu$m to 120 $\mu$m, and the height h be within the range of 200 $\mu$m to 400 $\mu$m. Further, as in FIG. 1 (b), in addition to the conical shape, the shape of the needle portions 4 may be so-called pencil-like, having a cylindrical column shape with a conical upper portion, or may also be a polyangular pyramid whose section is triangle, quadrangle, or the like.

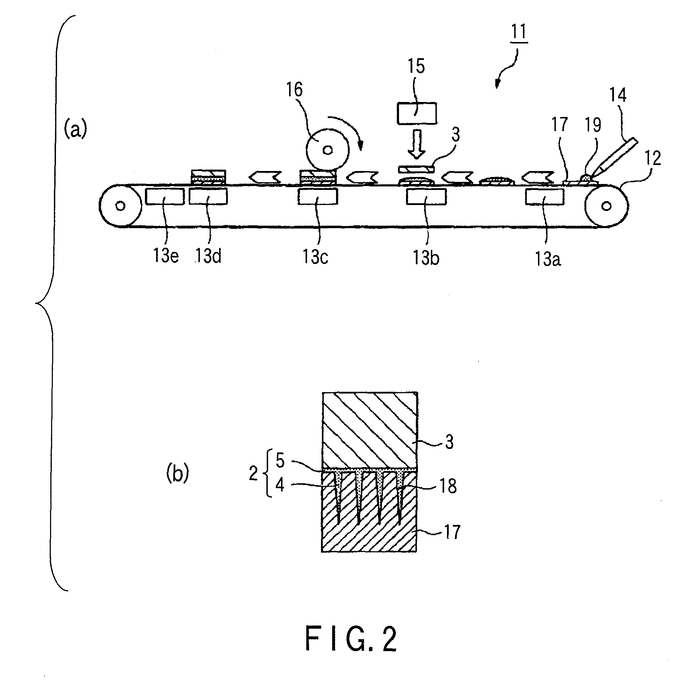

A method for producing the microneedle array 1 is explained with reference to FIG. 2. FIG. 2 (a) is a schematic diagram of a production apparatus 11 for the microneedle array 1 of this embodiment. The production apparatus 11 comprises a conveyor belt 12, heaters 13a to 13e provided along the transferring surface of the conveyor belt 12, a nozzle 14 provided in a predetermined position on the upstream portion of the conveyor belt, a substrate feeder 15 provided in a position downstream the nozzle 14, and a roll 16 provided downstream the substrate feeder 15 so as to pressurize the transferring surface of the conveyor belt 12.

First, on the conveyor belt 12, a mold 17 for forming the microneedle 2 is installed. As shown in FIG. 2 (b), the mold 17 is obtained by forming needle-forming portions 18 in a metal material by a known method such as photolithography, dry etching, or the like.

Next, a solution or a cutting block of medical-grade PLA 19, a product of Birmingham Polymers Inc., is fed onto the mold 17 from the nozzle 14. At this time, the temperature of the heater 13a is set at the melting point (hereinafter referred to as "Tm") of PLA 19 or higher.

As moving on the conveyor belt 12, the mold 17 is heated with the heater through the belt, whereby the PLA 19 is heated to the temperature range (°C) expressed by the equation (1) below and is spread out over the entire surface of the mold 17 to obtain the shape of the large number of needle portions 4 integrated at the sheet portion 5 (microneedle formation process). At this time, the temperature of the heater 13b is set at the Tm of PLA.

$$\text{Tm} + X \text{ (X is 2 or more and less than 50,}$$

$$\text{and preferably 2 or more and less than 10) ... (1)}$$

Subsequently, the mold 17 moves on the conveyor belt 12, and a substrate 3 is installed on the mold 17 from the substrate feeder 15. Although the substrate 3 is made of PMMA as mentioned above, a copolymer of butyl acrylate and methacrylate or the like is also suitable. Further, other plastic materials are also usable. In addition, alumina and metal, which are porous materials, may also be used. At this time, the temperature of the heater 13c is set at a temperature higher than the Tm of PLA by about 20°C, and is adjusted to be in the temperature range of the equation (1) when PLA reaches the heater 13c.

At least the processes from the feeding of PLA onto the mold to the formation of microneedle are to be performed under reduced pressure or vacuum.

The integrated substrate 3 and mold 17 are pressurized by the roll 16 and thus closely adhered, and, as shown in FIG. 2 (b), the substrate 3 and the sheet portion 5 are thermally fused (fusion process). At this time, the temperature of the roll 16 is set within the range of the above equation (1) (Tm is the melting point of PLA).

Subsequently, while moving on the conveyor belt 12, the temperature of the substrate 3 and the mold 17 are gradually lowered to about 70°C by the heaters 13d and 13e. After cooling, the substrate 3 integrated with the microneedle 2 is removed from the mold 17, and is punched into a desired shape, thereby giving the microneedle array 1 of this embodiment. To the surface of the needle portions 4 of the obtained microneedle array 1, insulin, estradiol, or a like hormone drug, nitroglycerin, or a like desired drug is applied in the form of a spray or a gel to form a drug layer. Thus, the microneedle array 1 can be used in transcutaneous administration of the drug.

According to the microneedle array 1 of the invention, the needle portions 4 are sufficiently adhered to the substrate 3 made of PMMA at the sheet portion 5, and thus have sufficient strength to resist plastic deformation even under a load of 5 kgf/cm$^2$ or less. Accordingly, when puncturing through the skin or a like biological surface, the needle portions satisfactorily reach the body tissue without plastic deformation. Further, the needle portions do not break in the body tissue. Even if they break, PLA that forms the needle portions 4 is decomposed in the body and disappears, and this thus causes no harm to the patient.

Further, because only the microneedle 2 is made of medical-grade PLA, as compared with the case where the entire microneedle array (i.e., the microneedle 2 and the substrate 3) is made of medical-grade PLA, the amount of the expensive medical-grade PLA to be used can be reduced by about 50% to about 80%. Accordingly, in comparison with conventional microneedle arrays, the manufacturing cost can be greatly reduced, while maintaining comparable performance.

Further, because the substrate 3 is made of flexible PMMA, it sufficiently follows the change in skin shape, and there is no need to worry about the separation of the microneedle 2 from the substrate 3, etc.

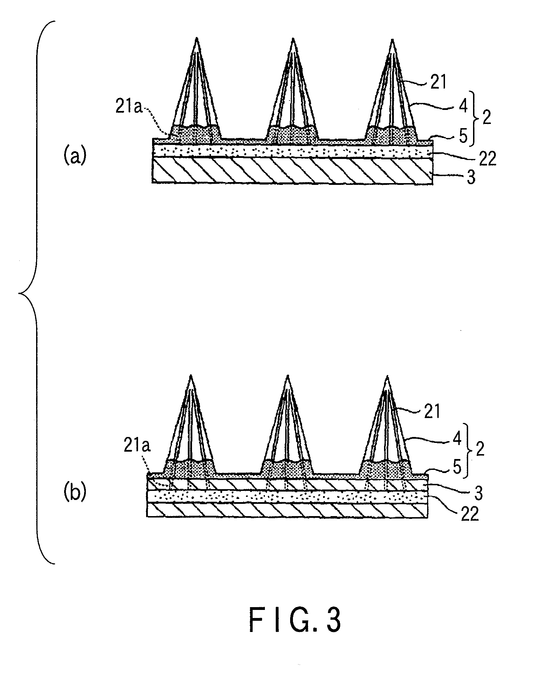

The microneedle 2 of this embodiment may have slots 21 formed on the surface thereof, as shown in FIG. 3 (a). According to such a structure, when a drug is applied to the surface of the microneedle 2, the drug is stored in the slots 21. This allows extension of the drug-releasing time and also enables more accurate control of drug release.

Further, as shown in FIG. 3 (a), communicating holes (let-out means) 21a each extending from a slot 21 and penetrating through the sheet portion 5 may also be provided. In this case, if a drug layer 22 comprising a polymer impregnated with a drug, for example, is provided in the gap between the sheet portion 5 and the substrate 3, the drug is let out from the drug layer 22 into the microneedle 2 through the communicating holes 21a and the release is thus continued even after the whole drug on the microneedle 2 is released. Accordingly, this allows further extension of the drug-releasing time. The drug layer 22 may be provided inside the substrate 3, as shown in FIG. 3 (b). In such a case, the microneedle array is structured so that communicating holes 21a extend inside the substrate 3 and communicate with the drug layer 22. However, in the case where the substrate 3 is formed using the above-mentioned porous material, the drug can be let out to the microneedle without communicating holes being formed. FIGS. 3 (a) and (b) each shows a section of a part of the substrate 3, the sheet portion 5, the drug layer 22, and the microneedles 2.

Although in the method for producing a microneedle array of this embodiment, a method in which production is performed while the mold and the substrate are moved has been explained, a microneedle array may be produced by heating a fixed mold, feeding PLA or a like biocompatible material thereto from micro-nozzles provided above the mold in correspondence with needle-forming portions to form a microneedle, and thermally fusing it with a substrate.

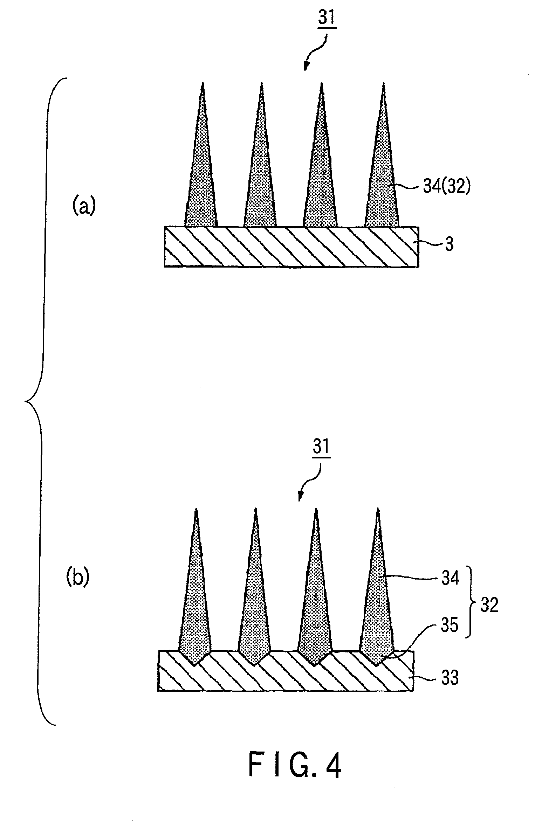

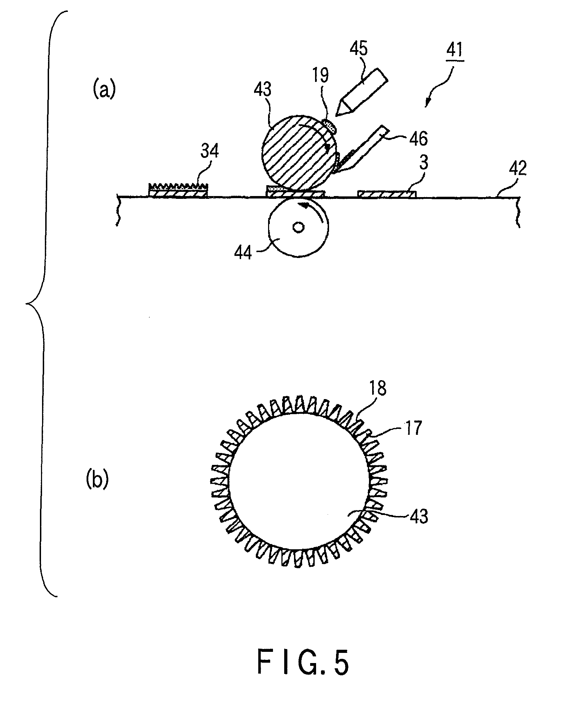

Next, a second embodiment of the invention is explained with reference to FIG. 4 and FIG. 5. The microneedle array 31 of this embodiment is different from the above first embodiment in that, as shown in FIG. 4 (a), the sheet portion 5 present in the microneedle 2 of the first embodiment is not present in a microneedle 32. The components common with the first embodiment are indicated with the same reference numerals, and duplicate explanations are omitted.

A method for producing the microneedle array 31 of this embodiment is explained with reference to FIG. 5. FIG. 5 (a) is a schematic diagram of a production apparatus 41 for the microneedle array 31 of this embodiment. The production apparatus 41 comprises a conveyor belt 42; a first roll 43 provided above the conveyor belt 42; a second roll 44 provided beneath the conveyor belt 42 to sandwich the conveyor belt together with the first roll 43, so that the conveyor belt can be pressurized; a nozzle 45 provided in a predetermined position above the first roll; and a knife edge 46 provided in a predetermined position beneath the nozzle 45, in such a manner that the tip thereof contacts the surface of the first roll. The first roll 43 is an imbricate roll, as shown in FIG. 5 (b), which has the above microneedle-forming mold 17 attached thereto in a many-sided manner with the needle-forming portions 18 facing the outer periphery.

First, a substrate 3 made of PC is installed on the conveyor belt 42. Next, medical-grade PLA 19, which has been heated to the melting temperature or higher, is fed to the surface of the first roll 43 from the nozzle 45. At this time, the first roll 43 has also been heated with a non-illustrated heater or the like to the PLA 19 melting temperature or higher. As the first roll 43 rotates, melted PLA 19 approaches the conveyor belt 42. During this process, the knife edge 46 removes excessive PLA 19 from the surface of the first roll 43. For this reason, the sheet portion 5 that is present in the microneedle 2 in the first embodiment is not formed in this embodiment.

When the substrate 3 moves on the conveyor belt 42, and is inserted between the first roll 43 and the second roll 44, PLA 19 melted on the surface of the first roll 43 is transferred to the surface of the substrate 3 to form microneedles 34, and, at the same time, the substrate 3 and the microneedles 34 are thermally fused. At this time, the second roll 44 is heated by a non-illustrated heater or the like to a temperature lower than the PLA 19 melting temperature by about 20°C.

After passing between the first roll 43 and the second rolls 44, the substrate 3 is naturally cooled by ambient air while moving on the conveyor belt 42. The thus-obtained substrate 3 having the microneedles 34 is punched into a desired shape and size, thereby giving the microneedle array 31 of this embodiment.

According to the microneedle array 31 of this embodiment, because the sheet portion 5 that is present in the first embodiment is not present in the microneedle 32, the amount of the medical-grade PLA to be used can be further reduced, thereby enabling reduction of manufacturing cost.

Although the microneedles 34 of this embodiment have a flat bottom, the microneedles 34 may each have an anchor portion 35 that digs into the substrate as shown in FIG. 4 (b). In such a case, performing production by the above method using a substrate 33 having fine asperities previously formed on the surface thereof can form a microneedle array provided with microneedles 34 having anchor portions 35.

Further, in the method for producing a microneedle array of this embodiment, although explained is the case where the substrate 3 is in the form of a sheet, the substrate may also be a continuous film. Further, by adjusting the distance between the knife edge 46 and the first roll 43, it is also possible to form a microneedle provided with a sheet portion, as in the first embodiment.

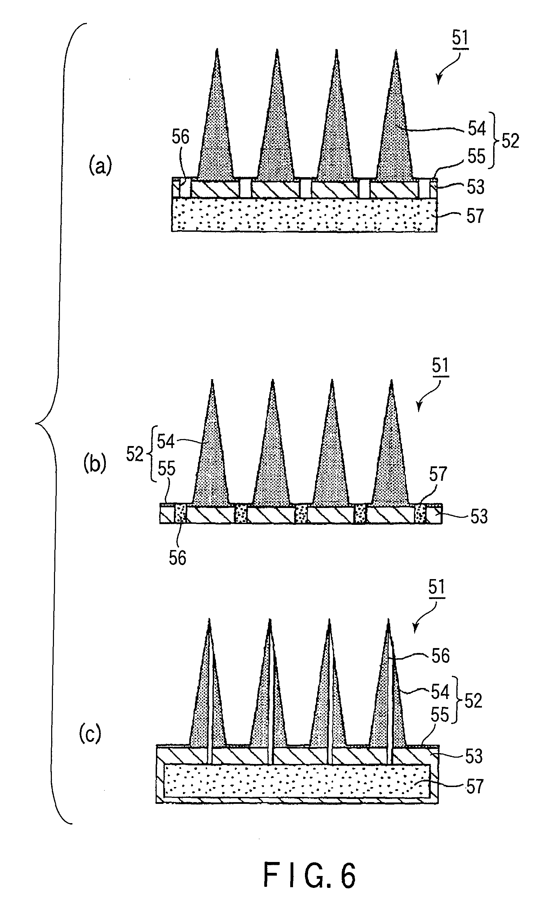

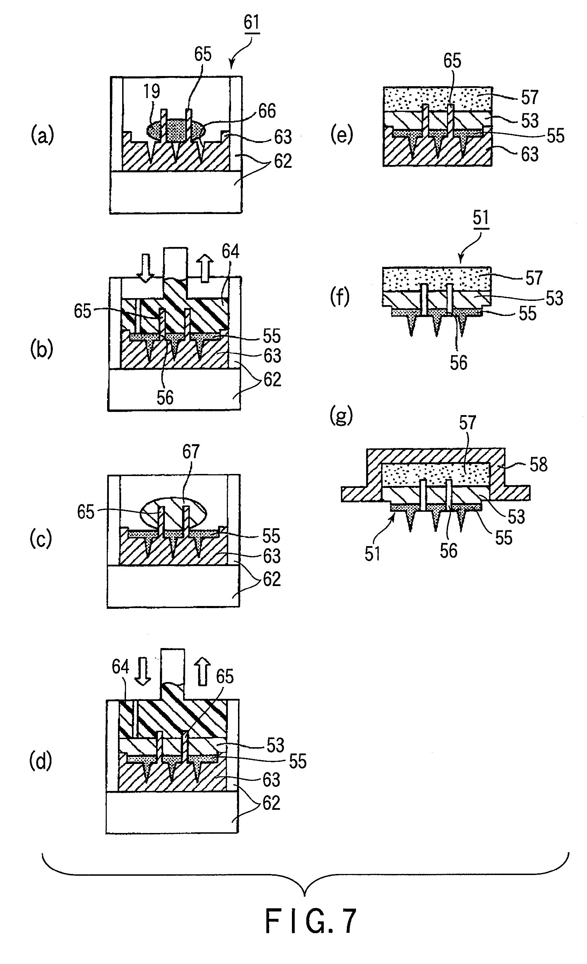

Next, a third embodiment of the invention is explained with reference to FIG. 6 and FIG. 7. A microneedle array 51 of this embodiment is different from the above embodiments, as shown in FIG. 6 (a), in that a large number of communicating holes 56 communicating with a substrate 53 are formed in a sheet portion 55, and that a drug layer 57 is provided beneath the substrate. The components common with the above embodiments are indicated with the same reference numerals, and duplicate explanations are omitted.

A method for producing the microneedle array 51 of this embodiment is explained with reference to FIG. 7. A production apparatus 61 for the microneedle array 51 comprises an outer frame 62, a mold 63 to be inserted into the outer frame 62, and a press plate 64. Unlike the above embodiments, the outer frame 62 and the mold 63 are previously formed into the shape and size of the microneedle array 51 to be produced.

The mold 63 is formed by machining a mold produced by almost the same method as that of the first embodiment into a desired shape and size, however, unlike the above mold 17, the mold 63 has a large number of protruding portions 65 formed thereon for forming the communicating holes 56 in the sheet portion 55.

First, as shown in FIG. 7 (a), the mold 63 is inserted into the outer frame 62, and a block of medical-grade PLA 19 is fed onto the mold 63. The block may have any shape, such as the shape of a sphere, a rectangular solid, a cylinder column, or the like. The mold 63 is heated with a non-illustrated heater, etc. PLA 19 is heated by the mold 63 to a temperature T°C expressed by the following equation (2), and is spread out in needle-forming portions 66 following the shape of the mold 63. The amount of PLA 19 is an amount to allow the PLA 19 to fill needle-forming portions 66 of the mold 63 and further form the sheet portion 55. Subsequently, as shown in FIG. 7 (b), a pressure of 50 MPa or more is applied by the press plate 64, then the temperature is lowered, and the press plate 64 is raised (microneedle formation process). At this time, the protruding portions 65 fit into the large number of holes provided in the press surface of the press plate 64. The resulting microneedle 52 has, in the sheet portion 55, a large number of communicating holes 56 formed by the protruding portions 65.

$$Tg + Y < T < Tm + 50 \text{ (Tg is the glass transition}$$
$$\text{temperature; and Y is 2 or more and less than 50,}$$
$$\text{and preferably 20 or more)} \qquad \ldots (2)$$

Subsequently, as shown in FIG. 7 (c), a block of PMMA 67 used as a material of the substrate 53 is fed onto the sheet portion 55. In place of PMMA 67, polyethylene (PE) or a like material may also be used. A material having a Tm comparable to or lower than that of the material of the microneedles 54 is preferably used. The amount of PMMA 67 is adjusted so that when the PMMA 67 is spread out uniformly over the sheet portion 55, the tips of the protruding portions 65 are not buried. Subsequently, while heating PMMA 67 to the temperature T calculated by the above equation (2), the press plate 64 applies a pressure of 50 MPa to compress PMMA 67 as shown in FIG. 7 (d), and the substrate 53 is thus formed (substrate formation and adhesion process).

After the press plate 64 is raised, the mold 63 is removed from the outer frame 62. Then, as shown in FIG. 7 (e), the drug layer 57 comprising a polymer impregnated with a desired drug is closely adhered onto the substrate 53. After the close adhesion, as shown in FIG. 7 (f), the substrate 53 is removed from the mold 63. This provides the microneedle array 51 of this embodiment, which has a large number of communicating holes 56 running through the sheet portion 55 and the substrate 53 and communicating with the drug layer 57. FIG. 7 (g) shows an end product formed of the microneedle array 51 incorporated with a patch member 58 coated with an adhesive material.

According to the microneedle array 51 of this embodiment, because of the communicating holes 56 formed in the sheet portion 55, the drug is released through the communicating holes 56 without being influenced by the behavior of the microneedles 54 in the body tissue, and thus, stably released into the body tissue through the holes formed in the biological surface by the puncture of the microneedles 54. Accordingly, drug release can be controlled more stably.

When the amount of charged drug is not large, it is also possible to form the drug layer 57 only in the communicating holes 56, as shown in FIG. 6 (b). Further, in the case where the protruding portions 65 of the mold 63 are provided in the needle-forming portions 66, the microneedles 54 can be formed into a hollow structure, as shown in FIG. 6 (c). In such a case, the drug can be administered into the body tissue more efficiently through the communicating holes 56 formed in the microneedles 54.

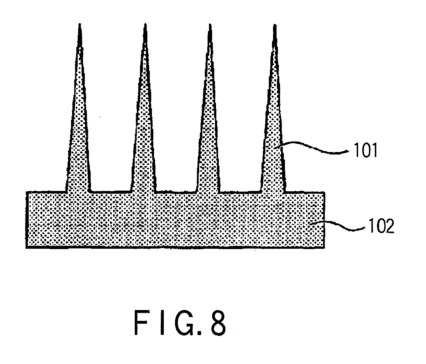

Although examples of a two-layer structure of a microneedle and a substrate have been explained above, the invention may be structured so that, as shown in FIG. 8, a microneedle 101 and a substrate 102 are formed in a single layer. Such an embodiment is explained hereinafter as a fourth embodiment of the invention.

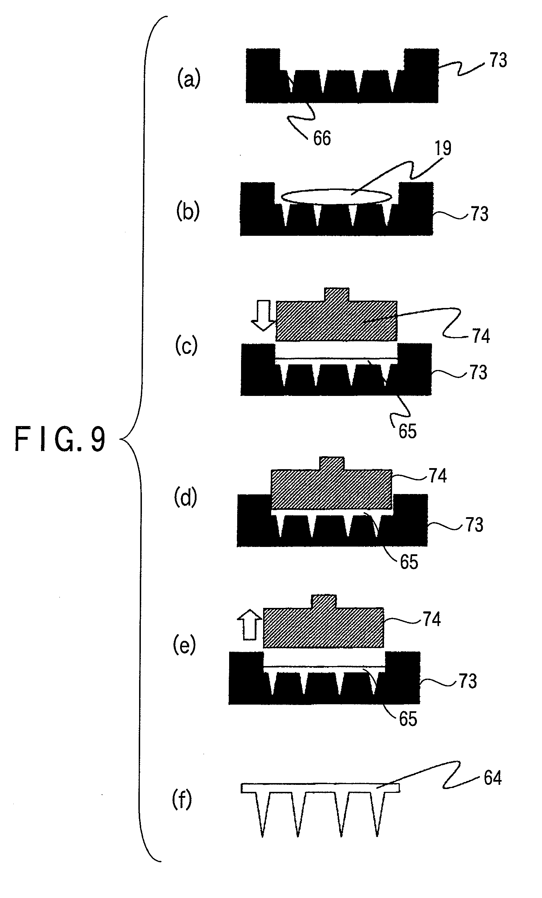

A method for producing a microneedle array of this embodiment is explained with reference to FIG. 9. First, as shown in FIG. 9 (b), medical-grade PLA 19 is fed onto a mold 73 under reduced pressure or vacuum. PLA 19 is heated, and, as shown in FIG. 9 (c), is spread out in needle-forming portions 66 following the shape of the mold 73. The amount of PLA 19 is an amount to allow the PLA 19 to fill the needle-forming portions 66 of the mold 73 and further form a sheet portion 65 with a thickness sufficient for the sheet portion 65 to serve as a substrate. Subsequently, as shown in FIG. 9 (d), a downward pressure is applied by a press plate 74, then the temperature is lowered to cause cooling and solidification, and the press plate 74 is raised, thereby giving a microneedle array 64.

According to the microneedle array 64 of this embodiment, because the microneedle array 64 is formed of a single layer, the number of manufacturing processes can be reduced, and the productivity can be improved.

As shown in the third embodiment, it is also possible to provide the mold with protruding portions to form communicating holes in the microneedle.

Embodiments of the invention have been explained thus far. However, the technical scope of the invention is not limited to the above embodiments, and various modifications may be made without deviating from the spirit of the invention.

For example, although the microneedle is made of medical-grade PLA in the above embodiments, insofar as the medical grade is satisfied, PLGA, chitin, chitosan, hyaluronic acid, collagen, glucose, cellulose, magnesium alloy, and other biocompatible materials may also be used. Further, the microneedle may also be made of a mixed material of the above biocompatible materials and the drug. In such a case, the drug is released by dissolution of the microneedle in the body tissue.

Further, although the substrate is made of PMMA in the above embodiments, as mentioned above, a copolymer of butyl acrylate and butyl methacrylate, polycarbonate, polyurethane, polypropylene, and other resin materials, metals, ceramics, and the like may also be used. In addition, PLA and like materials of a grade lower than the medical grade are also usable. In view of the conformability with the change in shape of the biological surface, the substrate is preferably made of a highly expansible resin material. Further, it is also possible that a plurality of layers made of the above various materials be integrated to form a substrate.

Further, although the substrate and the microneedle are adhered by thermal fusion in the above embodiments, they may be adhered by plasma welding. Further, although the substrate and the microneedle are formed by compression molding in the above embodiment, a common plastic molding technique, such as injection molding or the like, may be used instead to form the substrate and the microneedle.

Example 1

While vacuuming, PLA on a mold was heated to 210°C. The PLA was charged into needle-forming portions, and then cooled and solidified at normal temperature over 1 hour, thereby molding a microneedle array. For the molding of microneedles, the apparatus shown in FIG. 9 was used.

The molded microneedle array was removed, and the shape of the needle tip portions was observed. As a result, the needle tip portions were sharp, and the tip portions of all the microneedles on the microneedle array had the same shape.

Comparative Example 1

PLA on a mold was heated to 210°C without vacuuming. The PLA was charged into needle-forming portions, and then cooled and solidified at normal temperature over 1 hour, thereby molding a microneedle array. For the molding of microneedles, the apparatus shown in FIG. 9 was used.
The molded microneedle array was removed, and the shape of the needle tip portions was observed. As a result, rounded tip portions were observed in 80 needles out of every 800. The microneedles varied in shape and height.

Example 2

Chitin was dissolved in chloroform to give a resin fluid. The resin fluid was fed onto the mold, and vacuuming was performed while increasing the temperature to 60°C. After 1 hour, at the time when the organic solvent chloroform evaporated, the temperature was lowered to normal temperature, whereby only a chitin molded product was left on the mold. A chitin microneedle array was thus obtained. For the molding of microneedles, the apparatus shown in FIG. 9 was used.
The molded microneedle array was removed, and the shape of the needle tip portions was observed. As a result, the needle tip portions were sharp, and all the microneedle tip portions on the microneedle array had the same shape.

Reference Example 2

Chitin was dissolved in chloroform to give a resin fluid. The resin fluid was fed onto the mold, and charged into the mold while increasing the mold temperature to 60°C.
After 1 hour, the temperature was lowered to normal temperature, thereby molding a microneedle array. For the molding of the microneedle array, the apparatus shown in FIG. 9 was used.
The obtained microneedle array contained the organic solvent chloroform remaining therein, and thus was unusable for puncturing through a biological surface.

Example 3

Chitosan, a protein preparation, and water were mixed to give a resin fluid. The resin fluid was fed onto the mold, and the mold was heated to 40°C. Vacuuming was performed, and the mold was maintained at 40°C and left to stand for 1 hour in this state. Subsequently, the temperature was lowered to normal temperature, whereby only a chitosan molded product was left on the mold. A chitosan microneedle array was thus obtained. For the molding of the microneedle array, the apparatus shown in FIG. 9 was used.
The molded microneedle array was removed, and the shape of the needle tip portions was observed. As a result, the needle tip portions were sharp, and all the microneedle tip portions on the microneedle array had the same shape.

Comparative Example 3

Chitosan, a protein preparation, and water were mixed to give a resin fluid. The resin fluid was fed onto the mold. The mold was heated to 40°C and left to stand for 10 hours in this state. After 10 hours, the mold temperature was lowered to normal temperature, and molding was thus performed. For the molding of microneedles, the apparatus shown in FIG. 9 was used.
In Comparative Example 3, solidification into the microneedle array shape took time ten times longer than time required when vacuuming was included.

Industrial Applicability

The invention can be used as a microneedle array for medical applications.

Brief Description of Drawings

FIG. 1 is sectional views each schematically showing a microneedle array according to a first embodiment of the invention;

FIG. 2 shows a production method according to the same embodiment;

FIG. 3 is partial sectional views each schematically showing a modified example according to the same embodiment;

FIG. 4 is sectional views each schematically showing a microneedle array according to a second embodiment of the invention;

FIG. 5 shows a production method according to the same embodiment;

FIG. 6 is sectional views each schematically showing a microneedle array according to a third embodiment of the invention;

FIG. 7 shows a production method according to the same embodiment;

FIG. 8 is a sectional view schematically showing a microneedle array according to a fourth embodiment of the invention; and

FIG. 9 shows a production method according to the same embodiment.

Description of Reference Symbol

| | |
|---|---|
| 1, 31, 51, | 64: Microneedle array |
| 2, 32, 52: | Microneedle |
| 3, 33, 53: | Substrate |
| 4, 34, 54: | Microneedle |
| 17, 63, 73: | Mold |
| 19: | Polylactic acid (biocompatible material) |
| 21a: | Communicating hole (let-out means) |
| 22, | 57: Drug layer |

**Claims**

1.  A method for producing a microneedle, **characterized by** comprising:

    a feeding step of feeding a resin fluid to a forming mold having a needle-forming portion with an opening diameter of 50 to 200 $\mu$m and a depth of 100 to 500 $\mu$m;
    a charging step of charging the fed resin fluid into the needle-forming portion; and
    a solidifying step of cooling and solidifying the charged resin fluid,

    wherein the feeding step, the charging step, and the solidifying step are performed under reduced pressure or vacuum.

2.  A method for producing a microneedle, **characterized by** comprising:

    a feeding step of feeding a resin fluid to a forming mold having a needle-forming portion with an opening diameter of 50 to 200 $\mu$m and a depth of 100 to 500 $\mu$m;
    an overheating and melting step of fluidizing the fed resin to produce a resin fluid;
    a charging step of charging the resin fluid into the needle-forming portion; and
    a solidifying step of cooling and solidifying the charged resin fluid,

    wherein the feeding step, the overheating and melting step, the charging step, and the solidifying step are performed under reduced pressure or vacuum.

3.  The method for producing a microneedle according to claim 1 or 2, **characterized in that** the forming mold has two or more needle-forming portions.

4.  The method for producing a microneedle according to one of claims 1 to 3, **characterized in that** the forming mold has a protruding portion in an area other than the needle-forming portion.

5.  The method for producing a microneedle according to one of claims 1 to 3, **characterized in that** the forming mold has a protruding portion in the needle-forming portion.

6. The method for producing a microneedle according to one of claims 1 to 5, **characterized in that** the step of charging the resin fluid is performed by pressing the resin fluid.

7. The method for producing a microneedle according to one of claims 1 to 6, **characterized in that** in the step of charging the resin fluid, the resin fluid is placed also in an area on the forming mold other than the needle-forming portion.

8. The method for producing a microneedle according to one of claims 1 to 7, **characterized in that** the resin contains a biocompatible material.

9. The method for producing a microneedle according to claim 8, **characterized in that** the biocompatible material is a material containing one of polylactic acid, glycol lactic acid, chitin, chitosan, hyaluronic acid, collagen, glucose/ cellulose, and magnesium alloy.

10. A microneedle **characterized in that** it is produced by the producing method according to one of claims 1 to 9.

11. The microneedle according to claim 10, **characterized in that** the microneedle does not undergo plastic deformation under a load of 5 kg/cm$^2$ or less.

12. A method for producing a microneedle array, **characterized by** comprising:

a placing step of placing, after forming the microneedle by the producing method according to one of claims 1 to 9, a substrate such that it faces the needle-forming portion of the forming mold with the microneedle interposed therebetween; and
a stacking step of integrating the needle-forming portion and the substrate together.

13. The method for producing a microneedle array according to claim 12, **characterized in that** the microneedle and the substrate are made of different materials.

14. A microneedle array **characterized in that** it is manufactured by claim 12 or claim 13.

F I G . 1

(a)

(b)

F I G. 2

F I G. 3

## F I G. 4

(a)

(b)

FIG. 5

F I G. 6

F I G. 7

— 101

— 102

# FIG. 8

FIG.9

(a) 73 / 66

(b) 19 / 73

(c) 74 / 73 / 65

(d) 74 / 73 / 65

(e) 74 / 73 / 65

(f) 64

## INTERNATIONAL SEARCH REPORT

| | International application No. |
| --- | --- |
| | PCT/JP2007/072555 |

| A. CLASSIFICATION OF SUBJECT MATTER |
| --- |
| *A61M37/00*(2006.01)i, *B29C43/02*(2006.01)i, *B29C43/56*(2006.01)i |

According to International Patent Classification (IPC) or to both national classification and IPC

| B. FIELDS SEARCHED |
| --- |
| Minimum documentation searched (classification system followed by classification symbols) |
| A61M37/00, B29C43/02, B29C43/56 |

Documentation searched other than minimum documentation to the extent that such documents are included in the fields searched

| Jitsuyo Shinan Koho | 1922–1996 | Jitsuyo Shinan Toroku Koho | 1996–2008 |
| Kokai Jitsuyo Shinan Koho | 1971–2008 | Toroku Jitsuyo Shinan Koho | 1994–2008 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| X | WO 2006/121110 A1  (Fujikura Ltd.), 16 November, 2006 (16.11.06), Claims; Par. Nos. [0045] to [0055]; Figs. 8 to 10 (Family: none) | 1-14 |
| A | JP 2003-501162 A  (The Procter & Gamble Co.), 14 January, 2003 (14.01.03), Full text; all drawings & WO 2000/074765 A1    & US 6256533 B1 | 1-14 |
| A | WO 2005/082596 A1  (3M INNOVATIVE PROPERTIES CO.), 09 September, 2005 (09.09.05), Full text; all drawings & US 2007/0191761 A1    & JP 2007-523771 A | 1-14 |

☐ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 January, 2008 (24.01.08) | 05 February, 2008 (05.02.08) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**EP 2 090 331 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005503194 PCT